(19) **Europäisches Patentamt**
**European Patent Office**
**Office européen des brevets**

(11) **EP 4 483 991 A1**

(12) **EUROPEAN PATENT APPLICATION**

(43) Date of publication:
**01.01.2025  Bulletin 2025/01**

(21) Application number: **23181449.2**

(22) Date of filing: **26.06.2023**

(51) International Patent Classification (IPC):
**B01D 61/22** (2006.01)     **B01D 61/18** (2006.01)

(52) Cooperative Patent Classification (CPC):
**B01D 61/18; B01D 61/22; C07K 1/34;**
B01D 2311/16; B01D 2313/903; B01D 2315/10;
B01D 2315/16

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Sartorius Stedim Biotech GmbH
37079 Göttingen (DE)**

(72) Inventors:
• **STARK, Oliver**
  **37079 Göttingen (DE)**
• **AISSA, Tarek**
  **37079 Göttingen (DE)**
• **HELLING, Alexander**
  **37079 Göttingen (DE)**

(74) Representative: **Gottschald
Patentanwälte Partnerschaft mbB
Klaus-Bungert-Straße 1
40468 Düsseldorf (DE)**

(54) **METHOD FOR PERFORMING A TANGENTIAL FLOW FILTRATION ON A BIOMOLECULAR SOLUTION**

(57)    The invention relates to a method for performing a tangential flow filtration on a biomolecular solution, in particular for performing a single pass tangential flow filtration on a biomolecular solution, as part of a production process for biomolecules, wherein a tangential flow filtration arrangement (1) is provided, wherein the tangential flow filtration arrangement (1) comprises a tangential flow filtration module (2), wherein the tangential flow filtration is performed inside the tangential flow filtration module (2), wherein the tangential flow filtration module (2) comprises a filter (3), a feed input fluid line (4) for the biomolecular solution as a feed medium, a retentate output fluid line (6) for a retentate, a filtrate output fluid line (7) for a filtrate, and preferably a buffer input fluid line (5) for a buffer, wherein the tangential flow filtration arrangement (1) comprises at least two actuators (8) influencing the tangential flow filtration, wherein the tangential flow filtration arrangement (1) comprises a control module (10), wherein the control module (10) controls the tangential flow filtration by providing control signals to the actuators (8) and measuring at least two measured variables (11) describing the tangential flow filtration, wherein the control module (10) uses a process control to control the tangential flow filtration. It is proposed that the process control is a model predictive control (13), that the control module (10) inputs the measured variables (11) as input variables (14) into the model and derives at least two manipulated variables (15) as control signals for the actuators (8).

Fig. 3

**Description**

The present invention relates to a method for performing a tangential flow filtration on a biomolecular solution according to the general part of claim 1, to a control module according to claim 15 and to a tangential flow filtration arrangement according to claim 16.

The present teaching concerns filtration, in particular single pass filtration, in a biotechnological environment. Single pass tangential flow filtration (SPTFF) is a technique used in bioprocessing to separate and purify biomolecules, such as proteins, from mixtures. It is increasingly used in the production of biopharmaceuticals and other high-value biological products.

In SPTFF, a feed medium containing the biological molecules to be purified is passed through a filtration membrane under pressure. SPTFF can be performed through a tangential flow filtration module with a filter, usually a membrane. On a first side of the membrane and a first side of the module, the feed medium and optionally a buffer are fed into the module. On the second side of the module, after having passed along the membrane in tangential direction and partially through the membrane, the feed medium has separated into a retentate and a filtrate. The biomolecules may be part of either the retentate or the filtrate, depending on the specific process. SPTFF is well suited for concentration and purification of various components, including proteins like monoclonal antibodies and the like. For applications requiring buffer exchange or desalting of a solution, a buffer, sometimes also referred to as diafiltration medium, is added to the feed medium or retentate. This process is typically called diafiltration.

One of the main factors in efficiency of the SPTFF is the transmembrane pressure. Other relevant process variables are the concentration factor and the diafiltration factor.

Known single pass tangential flow filtration arrangements are sometimes controlled via, for example three, PID controllers. PID controllers are widely used for many types of control and usually behave well with less complex systems. Tangential flow filtrations for biomolecules often have constant set values that are set at the beginning of the filtration and often not changed during the filtration. They are usually not subject to abrupt changes or unexpected disturbance variables.

The control parameters of the PID controller may be adapted by a skilled user depending on the specific application, in particular the specific feed medium.

However, due to a variability between different customers and different batches and the slow changes of the system due to fouling and/or degradation of the membrane, a PID controller often needs at least some user supervision and a skilled user for the adaption of the PID. While such a control is not enough to solve the dynamic dependencies between the variables from a model-theoretic point of view, it has so far been used as a good approximation to support an operator.

A model predictive control or model predictive controller (MPC) is a control that uses a model to predict at least one future state and the corresponding outputs of a controlled system and takes this state and the outputs into account when deriving control signals to influence manipulated variables of the controlled system.

Model predictive controls are used in advanced control projects in refineries, the petrochemical industry, the chemical industry and some other industries. They are usually used for complex control systems with a high variability in the controlled variables and unknown disturbance variables. Model predictive controls provide a high flexibility for highly dynamic systems with coupled process variables and are able to deal with economic and technical changes and therefore varying operating points.

None of that is typical for a filtration in the bioprocessing field. There, the controlled variables and the process parameters in general are often kept relatively constant.

It is a challenge to improve the control in the known prior art.

The invention is based on the problem of improving the known methods such that a further optimization regarding the named challenge is reached.

The above-noted object is solved by the features of the characterizing part of claim 1.

The main realization of the present invention is that a model predictive control can be used for a biotechnological filtration to deal with changes between batches and customers. As a result, less user intervention is needed to adapt the control to new feed media or other changes in the system. Additionally, fouling and other deterioration effects of the filter, in particular membrane, are handled well by the model predictive control without user intervention. Further, the filtration process can be controlled in such a way that when one process variable is changed deliberately, only the value of this desired process variable is changed while leaving the values of the other process variables largely unaffected. As a bonus effect, the model can be used to determine a state of the filter and plan maintenance.

The proposed teaching concerns a method for performing a tangential flow filtration on a biomolecular solution, in particular for performing a single pass tangential flow filtration on a biomolecular solution, as part of a production process of the biomolecules, wherein a tangential flow filtration arrangement is provided, wherein the tangential flow filtration arrangement comprises a tangential flow filtration module, wherein the tangential flow filtration is performed inside the tangential flow filtration module, wherein the tangential flow filtration module comprises a filter, a feed input fluid line for the biomolecular solution as a feed medium, a retentate output fluid line for a retentate, a filtrate output fluid line for a filtrate, and preferably a buffer input fluid line for a buffer, wherein the tangential flow filtration arrangement comprises at least two actuators influencing the tangential flow filtration, wherein the tangential flow filtration arrangement comprises a control module, wherein the control module controls the tangential flow filtration by providing control signals to the actuators and measuring at least two measured variables describing the tangential flow filtration, wherein the control module uses a process control to control the tangential flow filtration.

In detail, it is proposed that the process control is a model predictive control, that the control module inputs the measured variables as input variables into the model and derives at least two manipulated variables as control signals for the actuators.

Claim 2 describes the preferred actuators for the tangential flow filtration arrangement and claim 3 the preferred manipulated variables.

In an embodiment according to claim 4, the controlled variables are set by a user and then preferably held constant. This may be the case for all controlled variables, emphasizing the simplicity of the process control in terms of changes during the process. Claim 5 names the preferred controlled variables.

Embodiments of claims 6 and 7 concern the model, here a state space model, of the model predictive control. The number of states of the model may be in a lower range (claim 7). The chosen states describe the physical properties of the filtration, chosen to achieve a less complex representation of the tangential flow filtration module based on the inputs and outputs and possible measurements outside the tangential flow filtration module.

According to claim 8 the moving horizon principle may be applied, ensuring quick reactions in case of unexpected changes and according to claim 9 the model predictive control may include limits for the manipulated and/or predicted variables. Including hard and soft limitations of the tangential flow filtration module in the model predictive control ensures compliance with those limits.

Claim 10 relates to embodiments of a cost function that have been found suitable for a stable control and a good adaption to new feed media.

A tangential flow filtration module usually does not include sensors inside the module. Further, even if sensors are present, they may not be able to measure all relevant parameters. Also, in particular flow sensors are prone to noise in their signals. An estimator may be used to mitigate some of those problems (claim 11). The estimator can be used to estimate any process variables inside or outside the tangential flow filtration module. The estimator may also estimate process variables that are also measured and in particular derive the degradation of the filter from the changes of those variables.

In an embodiment according to claim 12, the control module may choose one of multiple state space models at the beginning of a filtration to further adapt the model predictive control to the current feed medium and system configuration.

The state space model may also change over time to adapt to the filter degradation (claim 13).

Claim 14 relates to predictive maintenance based on the model predictive control. By analysing how the model predictive control changes over time, a good time for maintenance can be derived.

Another teaching according to claim 15, which is of equal importance, relates to a control module configured to use a state space model predictive control in a, in particular the proposed, method for performing a tangential flow filtration on a biomolecular solution, in particular for performing a single pass tangential flow filtration on a biomolecular solution, as part of a production process of the biomolecules.

All explanations given with regard to the proposed method are fully applicable.

Another teaching according to claim 16, which is of equal importance, relates to a tangential flow filtration arrangement configured to perform the proposed method.

All explanations given with regard to the proposed method and the proposed control module are fully applicable.

In the following, embodiments of the invention are explained with respect to the drawing. The drawing shows in

Fig. 1,    schematically a tangential flow filtration arrangement,

Fig. 2,    a simple overview over the process control and

Fig. 3,    a more complex overview over the model predictive control and its use.

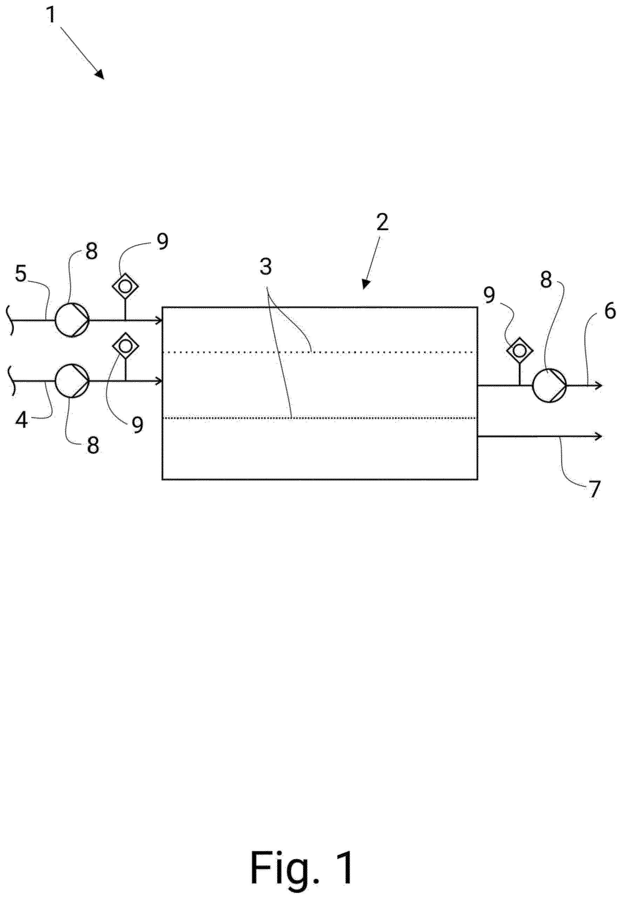

Fig. 1 shows a simplified tangential flow filtration arrangement 1 with a tangential flow filtration module 2. Here and preferably, the tangential flow filtration is a single pass tangential flow filtration, meaning that there is no recirculation of retentate flow back to the feed medium. The filtration may be continuous and/or a concentration and/or a diafiltration. The feed medium and the buffer are preferably added continuously.

The tangential flow filtration module 2 may be a single-use module or may comprise multiple components, for example a single-use filter 3 and a tube set. The filter 3 may comprise or be a membrane. As Fig. 1 shows, the tangential flow filtration module 2 may comprise one or two inlets, one connected to a feed input fluid line 4 and optionally one connected to a buffer input fluid line 5. The tangential flow filtration module 2 may further comprise two outlets connected to a retentate output fluid line 6 and a filtrate output fluid line 7. Actuators 8, in particular pumps or valves, and/or sensors 9 may be present in or at any one or any combination of the fluid lines.

With the shown tangential flow filtration module 2, a buffer exchange can be performed. Here, two membranes are present, dividing the tangential flow filtration module 2 into three chambers or channels. In the shown embodiment the top chamber for the buffer does not have an output fluid line such that the buffer can only exit the module through the filter 3. The shown bottom chamber for the filtrate does not have an input fluid line. Naturally, the terms top and bottom are only related to the figure and not necessarily to an actual orientation.

Proposed is a method for performing a tangential flow filtration on a biomolecular solution, in particular for performing a single pass tangential flow filtration on a biomolecular solution, as part of a production process for biomolecules.

The biomolecules may be small molecules, in particular proteins like antibodies, or other molecules produced in a bioprocess, in particular produced by living cells. Filtrations during the production of biomolecules typically need to meet regulatory standards during execution and control. The biomolecules need to be subjected to a controlled environment without leaving set boundaries to ensure the quality of the biomolecules.

Here and as shown in Fig. 1, a tangential flow filtration arrangement 1 is provided. The tangential flow filtration arrangement 1 comprises a tangential flow filtration module 2. The tangential flow filtration is performed inside the tangential flow filtration module 2. Usually, the feed medium is pressurized and pumped into the tangential flow filtration module 2. The resulting transmembrane pressure drives the liquid and small particles through the membrane while the bigger particles are unable to pass through the membrane. This separates the feed medium into a retentate and a filtrate. If, as preferably, a buffer is applied, the liquid of the feed medium may be exchanged for the buffer, for example to adjust the salt concentration for a subsequent process step such as a chromatography.

The tangential flow filtration module 2 comprises a filter 3, a feed input fluid line 4 for the biomolecular solution as a feed medium, a retentate output fluid line 6 for a retentate, a filtrate output fluid line 7 for a filtrate, and, here and preferably, a buffer input fluid line 5 for a buffer. Here, the filter 3 is a membrane inside the tangential flow filtration module 2.

The tangential flow filtration arrangement 1 comprises at least two actuators 8 influencing the tangential flow filtration, here three pumps.

The tangential flow filtration arrangement 1 comprises a control module 10. The control module 10 controls the tangential flow filtration by providing control signals to the actuators 8 and measuring at least two measured variables 11 describing the tangential flow filtration. The control signals may be direct control signals like a PWM voltage applied to a pump. The control signals may also be indirect control signals, for example set values 12 for a further control system. The same is true for the measured variables 11. In particular, a general process control system may be provided, and the control module 10 may communicate with that general process control system to receive the measured variables 11 and send the control signals. The control module 10 uses a process control to control the tangential flow filtration.

Essential is, that the process control is a model predictive control 13. The control module 10 inputs the measured variables 11 as input variables 14 into the model and derives at least two manipulated variables 15 as control signals for the actuators 8. Here and preferably, the process control works in real time.

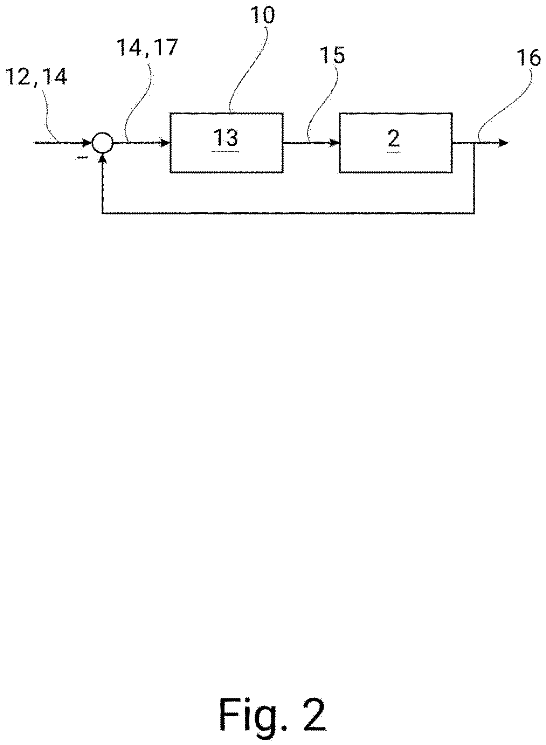

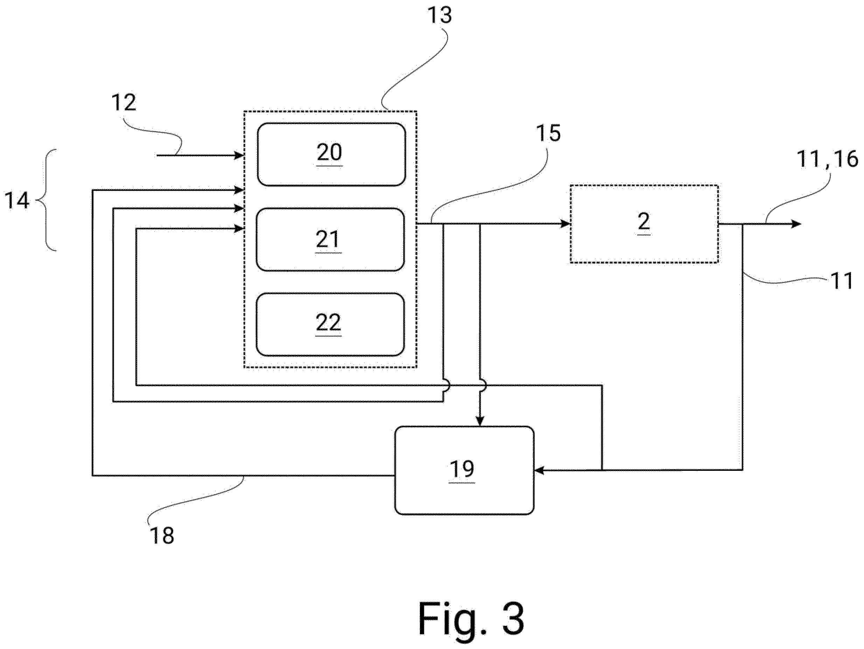

A general overview over the process control is shown in Fig. 2. Fig. 2 is meant to give an overview over the used terminology. On the left side, the input variables 14 and set values 12 are shown. Input variables 14 are everything that is fed into the model, in particular, the set values 12 are also set values 12 of some input variables 14. The current values of the manipulated variables 16 are fed back onto the input variables 14 to derive errors 17 as is known from the art. It should be noted that in Fig. 3, the errors 17 are derived inside the model predictive control 13, if necessary. The errors 17 are used as input values for input variables 14. The control module 10 derives values for the manipulated variables 15 and applies them onto the tangential flow filtration module 2 by controlling the actuators 8. The current values of the manipulated variables 16 are shown as an output in Fig. 2. It should be understood that in the preferred example, the controlled variables themselves are not measured but either estimated or derived from the measured variables 11.

Fig. 3 shows a more detailed explanation of the preferred model predictive control 13. The model received set values 12, feedback of the last output manipulated variables 15 if the model does not save those and needs them, measured variables 11 and/or yet to be explained estimated values 18 from an estimator 19.

According to one embodiment and as already partly explained, it is proposed, that the tangential flow filtration arrangement 1 comprises at least one actuator 8, in particular a pump, acting on the feed input fluid line 4 and/or at least one actuator 8 acting on the retentate output fluid line 6 and/or at least one actuator 8 acting on the buffer input fluid line 5. Additionally or alternatively, the tangential flow filtration arrangement 1 comprises at least one sensor 9, in particular at least one pressure sensor 9 and/or at least one flow sensor 9, at the feed input fluid line 4 and/or at least one sensor 9, in particular at least one pressure sensor 9 and/or at least one flow sensor 9, at the retentate output fluid line 6 and/or at least one sensor 9, in particular at least one pressure sensor 9 and/or at least one flow sensor 9, at the buffer input fluid line 5. Additionally or alternatively, an actuator 8, in particular pump, and/or a flow sensor 9 may be present at the filtrate output fluid line 7.

The manipulated variables 15 may comprise a speed of the feed pump and/or a speed of the retentate pump and/or a speed of the buffer pump. The manipulated variables 15 may also comprise a pressure of the feed and/or of the retentate and/or of the buffer and/or a volume flow rate of the feed and/or of the retentate and/or of the buffer. The pressure and/or the volume flow rate may be manipulated indirectly, for example via a fast working PID controller that receives the set pressure and pressure sensor 9 signals and controls the speed of the respective pump.

Here and preferably, the manipulated variables 15 are the speed of the feed pump and of the retentate pump and of the buffer pump. Instead of a retentate pump or in addition thereto, a filtrate pump may be present. Everything explained for the retentate pump may be true for the filtrate pump instead or additionally.

The number of controlled variables may be rather small. Here and preferably, the controlled variables are the transmembrane pressure, the concentration factor and the diafiltration volume. The term "transmembrane pressure" also comprises generally a "transfilter pressure". Generally it is preferred, that the process control receives, in particular from a user, a set value 12 for at least one, preferably at least two, more preferably at least three, and/or at most five, more preferably at most 4, controlled variables controlled by the process control. The user may enter the set values 12 into the general control system mentioned above. The named quantities of controlled variables are preferred embodiments independently of the source of the set values 12. Additionally or alternatively, the controlled variables may comprise a conductivity and/or a pH value and/or any concentration.

The controlled variables may be constant during the filtration. Here and preferably, the controlled variables are held constant until the control module 10 receives a new set value 12 from the user.

It may be the case that least one, preferably all, controlled variables are not linearly dependent on the input variables 14 and/or the manipulated variables 15, and/or, that the number of controlled variables is equal to the number of actuators 8.

According to one embodiment it is proposed, that the controlled variables comprise or consist of a transmembrane pressure, in particular an average transmembrane pressure, and/or a concentration factor, in particular a ratio of feed volume flow rate to retentate volume flow rate, and/or a diafiltration ratio, in particular a ratio of buffer volume flow rate to feed volume flow rate.

Turning now towards the model, it is preferred, that the model predictive control 13 comprises a state space model 20 with state variables. Preferably, the state space model 20 describes the influence of the actuators 8 on the state of the tangential flow filtration module 2.

An exemplary state space model 20 may be of the form

$$\Delta x/\Delta t = Ax + Bu \text{ and } y = C(x)$$

with $\Delta$ representing the derivate in a continuous or numerically approximated manner or the difference between cycles of the prediction of the model predictive control 13. x is the vector of state variables. A is a matrix representing the behavior and interdependencies of the state variables. B represents the influence of the manipulated variables 15, named u, on the state variables.

y represents the controlled variables and C is the transformation matrix transforming the state variables into the controlled variables. It is preferably the case, that at least one, in particular each, controlled variable is derived from at least two state variables. This will usually mean that the controlled variables are not linearly dependent on any one state variable. The state space model 20 may be time invariant.

According to one embodiment it is proposed, that the state space model 20 has at least 3, preferably at least 6, more preferably at least 9, more preferably at least 12, and/or at most 30, preferably at most 24, more preferably at most 15, more preferably at most 12, state variables, and/or, that the state space model 20 comprises first order differential equations, in particular for each state variable, preferably exactly one for each state variable, and/or, that the state space model 20 does not comprise differential equations of an order higher than one, preferably, that the state variables include a pump velocity, in particular rotational velocity, and/or a flow rate of the feed pump and/or the retentate pump and/or the buffer pump and/or a volume flow rate of the feed and/or the retentate and/or the buffer and/or one or more, preferably all, pressure influences of the pumps on the, in particular mean, feed and retentate pressure.

Here it may be the case that the twelve state variables are the three pump velocities, three volume flow rates derived from the pump velocities and six pressure variables describing the pressure influence of each channel on the feed and retentate pressure inside the tangential flow filtration module 2, each derived from the volume flow rate. As the pumps are located outside the tangential flow filtration module 2, but the transmembrane pressure is preferably the mean pressure difference between the feed and the retentate side, the pressure is here and preferably derived by modeling the influences of the pumps on the pressure inside the tangential flow filtration module 2. The manipulated variables 15 are the pump velocities. This state space model 20 is a representation with low complexity. By superimposing the pressure influences of the different pumps, the model does not need a high calculation effort for numerical solving. The controlled variables can then be derived from the state variables.

Looking at the prediction, it may be the case that in a current cycle the model predictive control 13 predicts manipulated variables 15 for a prediction horizon of several cycles based on a current state, that the first predicted manipulated variables 15 are used as the control signals for the current cycle, that a new prediction cycle is started for the next cycle and preferably, that during the prediction the manipulated variables 15 are only changed during a control horizon of a number of cycles smaller than the prediction horizon. This embodiment is an implementation of the moving horizon principle.

It is also preferred, that the model predictive control 13 includes limits for the predicted variables and/or the manipulated variables 15. Those limits may include the physical laws and physical limits the tangential flow filtration module 2 faces. The limits may also include soft limits defined by a user, for example for regulation or economic purposes.

To derive the values for the manipulated variables 15, the model predictive control 13 may use an optimizer 21 to derive the manipulated variables 15.

Preferably, the optimizer 21 uses a cost function 22. The cost function 22 may include a term dependent on the deviation between the set value 12 and the current value in one or more prediction cycles, preferably the squared deviations of the calculated difference between the set value 12 and the current value of the manipulated variables 16 for the prediction cycles, in particular for each prediction cycle. This term makes sure that the manipulated variables 15 quickly reach the set value 12.

The cost function 22 may further include a term dependent on the changes of the manipulated variables 15 during one or more prediction cycle, preferably squared deviations of the changes of the manipulated variables 15 over the prediction cycles, in particular all prediction cycles. This term ensures low overshooting.

The cost function 22 may further include a term dependent on the deviations of the state variables over between at least two prediction cycles, preferably squared deviations of the changes of the system states during the prediction cycles. This term ensures that no abrupt changes are implemented which are physically impossible or stress the tangential flow filtration arrangement 1 or the biomolecular solution.

The cost function 22 may be not dependent on the point in time of the prediction cycles. A time invariant cost function 22 is well suited for a model with a good prediction over a longer time and few unexpected deviations. This is preferably the case here.

As already touched above, an estimator 19 may be used to estimate at least one input variable 14. Preferably, the estimated variable is input into the state space model 20.

Further it is possible, that the predicted variable is compared with the measured variable 11 to detect changes in the filter 3. This embodiment is interesting for predictive maintenance. By generally monitoring the behavior of the state space model 20 and changes in this behavior, conclusions about the state of the filter 3, in particular the degradation of the membrane, can be drawn.

It is also possible, that the control module 10 uses the detected changes to change the state space model 20. Automatically adapting the, in particular time invariant, state space model 20 allows keeping the state space model 20 up to date with the slow changes in the filter 3. A use case of the estimator 19 may be to estimate a measured variable 11, in particular with a model designed to be similar to the state space model 20. If the estimate starts deviating from the measured value, this may indicate a change in the filter 3.

A further use of the estimator 19 is to reduce noise in a measured variable 11. In particular flow data may have a lot of noise. As an alternative to filters, an estimator 19 may be able to derive a good estimate of the correct value.

According to one embodiment it is proposed, that the control module 10, in particular using the estimator 19, chooses one of multiple predefined state space models 20 as the state space model 20 to fit the current tangential flow filtration, in particular the current feed medium, preferably at the beginning of the tangential flow filtration. For that, multiple standard model may be saved in the control module 10. The standard models may include criteria for choosing one of them.

As mentioned in a preferred embodiment, the state space model 20 is time invariant. Alternatively, the state space model 20 may be, in particular slowly, time variant and adapted to the changes in the filter 3 over time. Further alternatively, the control module 10 may adapt the state space model 20 over time based on the measured variables 11, in particular as explained above. Preferably, the control module 10 compares the predicted manipulated variables 15 of previous cycles to the measured variables 11 and adapts the state space model 20 based on the deviations.

According to one embodiment it is proposed, that the changes of the state space model 20 are used to plan and/or predict a maintenance, in particular automatically.

Another teaching which is of equal importance relates to a control module 10 configured to use a model predictive control 13 in a method for performing a tangential flow filtration on a biomolecular solution, in particular for performing a single pass tangential flow filtration on a biomolecular solution, as part of a production process for biomolecules, in particular according to the proposed method. All explanations given with regard to the proposed method are fully applicable. In particular, the control module 10 may be adapted to perform any of the method steps described with regard to the control module 10.

Another teaching which is of equal importance relates to a tangential flow filtration arrangement 1 configured to perform the proposed method. All explanations given before are fully applicable.

**Claims**

1. Method for performing a tangential flow filtration on a biomolecular solution, in particular for performing a single pass tangential flow filtration on a biomolecular solution, as part of a production process for biomolecules,

wherein a tangential flow filtration arrangement (1) is provided, wherein the tangential flow filtration arrangement (1) comprises a tangential flow filtration module (2), wherein the tangential flow filtration is performed inside the tangential flow filtration module (2), wherein the tangential flow filtration module (2) comprises a filter (3), a feed input fluid line (4) for the biomolecular solution as a feed medium, a retentate output fluid line (6) for a retentate, a filtrate output fluid line (7) for a filtrate, and preferably a buffer input fluid line (5) for a buffer, wherein the tangential flow filtration arrangement (1) comprises at least two actuators (8) influencing the tangential flow filtration, wherein the tangential flow filtration arrangement (1) comprises a control module (10), wherein the control module (10) controls the tangential flow filtration by providing control signals to the actuators (8) and measuring at least two measured variables (11) describing the tangential flow filtration,

wherein the control module (10) uses a process control to control the tangential flow filtration, **characterized in**

**that** the process control is a model predictive control (13), that the control module (10) inputs the measured variables (11) as input variables (14) into the model and derives at least two manipulated variables (15) as control signals for the actuators (8).

2. Method according to claim 1, **characterized in that** the tangential flow filtration arrangement (1) comprises at least one actuator (8), in particular a pump, acting on the feed input fluid line (4) and/or on the retentate output fluid line (6) and/or on the buffer input fluid line (5), and/or, that the tangential flow filtration arrangement (1) comprises at least one sensor (9), in particular pressure sensor (9) and/or flow sensor (9), at the feed input fluid line (4) and/or at the retentate output fluid line (6) and/or at the buffer input fluid line (5).

3. Method according to claim 2, **characterized in that** the manipulated variables (15) comprise a speed of the feed pump and/or the retentate pump and/or the buffer pump and/or a pressure and/or volume flow rate of the feed and/or retentate and/or buffer, preferably a speed of the feed pump and the retentate pump and the buffer pump.

4. Method according to one of the preceding claims, **characterized in that** the process control receives, in particular from a user, a set value (12) for at least one, preferably at least two, more preferably at least three, and/or at most five, more preferably at most 4, controlled variables controlled by the process control, in particular held constant until receiving a new set value (12) from a user, that at least one, preferably all, controlled variables are not linearly dependent on the input variables (14) and/or the manipulated variables (15), and/or, that the number of controlled variables is equal to the number of actuators (8).

5. Method according to one of the preceding claims, **characterized in that** the controlled variables comprise or consist of a transmembrane pressure, in particular an average transmembrane pressure, and/or a concentration factor, in particular a ratio of feed volume flow rate to retentate volume flow rate, and/or a diafiltration ratio, in particular a ratio of buffer volume flow rate to feed volume flow rate.

6. Method according to one of the preceding claims, **characterized in that** the model predictive control (13) comprises a state space model (20) with state variables, preferably, that the state space model (20) describes the influence of the actuators (8) on the state of the tangential flow filtration module (2), and/or, that at least one, in particular each, controlled variable is derived from at least two state variables.

7. Method according to one of the preceding claims, **characterized in that** the state space model (20) has at least 3, preferably at least 6, more preferably at least 9, more preferably at least 12, and/or at most 30, preferably at most 24, more preferably at most 15, more preferably at most 12, state variables, and/or, that the state space model (20) comprises first order differential equations, in particular for each state variable, preferably exactly one for each state variable, and/or, that the state space model (20) does not comprise differential equations of an order higher than one, preferably, that the state variables include a pump velocity, in particular rotational velocity, and/or a flow rate of the feed pump and/or the retentate pump and/or the buffer pump and/or a volume flow rate of the feed and/or the retentate and/or the buffer and/or one or more, preferably all, pressure influences of the pumps on the, in particular mean, feed and retentate pressure.

8. Method according to one of the preceding claims, **characterized in that** in a current cycle the model predictive control (13) predicts manipulated variables (15) for a prediction horizon of several cycles based on a current state, that the first predicted manipulated variables (15) are used as the control signals for the current cycle, that a new prediction cycle is started for the next cycle, preferably, that during the prediction the manipulated variables (15) are only changed during a control horizon of a number of cycles smaller than the prediction horizon.

9. Method according to one of the preceding claims, **characterized in that** the model predictive control (13) includes limits for the predicted variables and/or the manipulated variables (15).

10. Method according to one of the preceding claims, **characterized in that** the model predictive control (13) uses an optimizer (21) to derive the manipulated variables (15), preferably, that the optimizer (21) uses a cost function (22) including squared deviations of the calculated difference between the set value (12) and the current value of the manipulated variables (16) for the prediction cycles and/or including squared deviations of the changes of the manipulated variables (15) over the prediction cycles and/or including squared deviations of the changes of the state variables during the prediction cycles and/or a cost function (22) not dependent on the point in time of the prediction cycles.

11. Method according to one of the preceding claims, **characterized in that** an estimator (19) is used to estimate at least one input variable (14), preferably, that the estimated variable is input into the state space model (20) and/or that the predicted variable is compared with the measured variable (11) to detect changes in the filter (3), more preferably, that the control module (10) uses the detected changes to change the state space model (20).

12. Method according to claims 6 to 11, **characterized in that** the control module (10), in particular using the estimator (19), chooses one of multiple predefined state space models (20) as the state space model (20) to fit the current tangential flow filtration, in particular the current feed medium, preferably at the beginning of the tangential flow filtration.

13. Method according to one of claims 6 to 12, **characterized in that** the state space model (20) is time invariant, or, that the state space model (20) is time variant and adapted to the changes in the filter (3) over time, or, that the control module (10) adapts the state space model (20) over time based on the measured variables (11), preferably, that the control module (10) compares the predicted manipulated variables (15) of previous cycles to the measured variables (11) and adapts the state space model (20) based on the deviations.

14. Method according to one of claims 11 to 13, **characterized in that** the changes of the state space model (20) are used to plan and/or predict a maintenance, in particular automatically.

15. Control module configured to use a model predictive control (13) in a method for performing a tangential flow filtration on a biomolecular solution, in particular for performing a single pass tangential flow filtration on a biomolecular solution, as part of a production process for biomolecules, in particular according to one of the preceding claims.

16. Tangential flow filtration arrangement configured to perform the method according to one of claims 1 to 14.

# Fig. 1

Fig. 2

Fig. 3

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | THAKUR GARIMA ET AL: "Implementing PAT for single-pass tangential flow ultrafiltration for continuous manufacturing of monoclonal antibodies", JOURNAL OF MEMBRANE SCIENCE, ELSEVIER BV, NL, vol. 613, 18 July 2020 (2020-07-18), XP086250450, ISSN: 0376-7388, DOI: 10.1016/J.MEMSCI.2020.118492 [retrieved on 2020-07-18] | 1-9,11, 13-16 | INV. B01D61/22 B01D61/18 |
| Y | * Title and Abstract; Section 1. page 2, right col., 3rd par; section 2.3. 1st, 2nd and 3rd par.; section 2.5. 1st par.; section 3.1, page 5, 2nd and 3rd par. and last par.; section 3.2; section 3.3. 2nd par. and section 3.3.1., 3.3.3. and 3.3.4.; section 3.3. 2nd par.;section 4, 1st par, page 11, left column; figures 2, 5C, 8 * | 10,12 | |
| Y | HUTER MAXIMILIAN J. ET AL: "Model-Based Design and Process Optimization of Continuous Single Pass Tangential Flow Filtration Focusing on Continuous Bioprocessing", PROCESSES, vol. 7, no. 6, 28 May 2019 (2019-05-28), page 317, XP093008705, DOI: 10.3390/pr7060317 * Abstract, page 2 l. 5-6 and page 3 l. 5-10, section 5. * | 10 | TECHNICAL FIELDS SEARCHED (IPC)  B01D |
| Y | WO 2021/205479 A2 (TATA CONSULTANCY SERVICES LTD [IN]) 14 October 2021 (2021-10-14) * claim 1 * | 10 | |

-----

-----

-----

-/--

The present search report has been drawn up for all claims

| Place of search | Date of completion of the search | Examiner |
|---|---|---|
| Munich | 13 October 2023 | Williams, Jennifer |

EPO FORM 1503 03.82 (P04C01)

page 1 of 2

Europäisches
Patentamt

European
Patent Office

Office européen
des brevets

# EUROPEAN SEARCH REPORT

Application Number

EP 23 18 1449

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/139290 A1 (BUDDHIRAJU VENKATA SUDHEENDRA [IN] ET AL) 4 May 2023 (2023-05-04) | 1-5, 8-11,15, 16 | |
| Y | * paragraphs [0017], [0019], [0020], [0048], [0058] – [0061]; claim 1; figure 3 * | 10,12 | |
| | ----- | | |
| X | THAKUR GARIMA ET AL: "Process Analytical Technology (PAT) Implementation for Membrane Operations in Continuous Manufacturing of mAbs: Model-Based Control of Single-Pass Tangential Flow Ultrafiltration", THE AAPS JOURNAL, SPRINGER INTERNATIONAL PUBLISHING, CHAM, vol. 24, no. 4, 13 July 2022 (2022-07-13), XP037902307, DOI: 10.1208/S12248-022-00731-Z [retrieved on 2022-07-13] | 1-9,11, 13,15,16 | |
| Y | * Title and Abstract; Introduction, page 2, right col, 2nd par and page 2, left col. 3rd par.; Modelling approach, page 4 right col. 2nd par, and page 5, left col. 1st and 2nd par. * | 10,12 | TECHNICAL FIELDS SEARCHED (IPC) |
| | ----- | | |

The present search report has been drawn up for all claims

| Place of search | Date of completion of the search | Examiner |
|---|---|---|
| Munich | 13 October 2023 | Williams, Jennifer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 1449

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-2023

| Patent document cited in search report | | Publication date | Patent family member(s) | | Publication date |
|---|---|---|---|---|---|
| WO 2021205479 | A2 | 14-10-2021 | EP | 4133340 A2 | 15-02-2023 |
| | | | US | 2023104214 A1 | 06-04-2023 |
| | | | WO | 2021205479 A2 | 14-10-2021 |
| US 2023139290 | A1 | 04-05-2023 | EP | 4173697 A1 | 03-05-2023 |
| | | | US | 2023139290 A1 | 04-05-2023 |

EPO FORM P0459

For more details about this annex : see Official Journal of the European Patent Office, No. 12/82